# EUROPÄISCHE PATENTSCHRIFT

(11) **EP 1 610 695 B1**
(45) Veröffentlichungstag und Bekanntmachung des Hinweises auf die Patenterteilung: **20.09.2006**
(21) Anmeldenummer: 05736140.4
(22) Anmeldetag: 20.04.2005
(51) Int. Cl.: A61B 17/32, A61B 17/28

(54) **CHIRURGISCHES INSTRUMENTENSYSTEM**
SURGICAL INSTRUMENT SYSTEM
SYSTEME D'INSTRUMENT CHIRURGICAL

(30) Priorität: 30.04.2004 DE 102004021713
(43) Veröffentlichungstag der Anmeldung: 04.01.2006
(73) Patentinhaber: Karl Storz GmbH & Co. KG, 78532 Tuttlingen (DE)
(72) Erfinder: DOLL, Frank, 78607 Talheim (DE); WALTER, Christian, 78576 Emmingen-Liptingen (DE); RÖSCH, Walter, 78166 Donaueschingen (DE)
(74) Vertreter: Hofmeister, Frank
(86) Internationale Anmeldenummer: PCT/EP2005/004196
(87) Internationale Veröffentlichungsnummer: WO 2005/104966

(56) Entgegenhaltungen:
- EP-A- 0 621 008
- DE-U1- 20 204 691
- US-A- 5 488 958
- US-A- 6 156 049
- US-B1- 6 468 228

## Beschreibung

Die Erfindung betrifft ein chirurgisches Instrumentensystem, umfassend einen Morcellator mit einem hohlen Schaft, an dessen distalem Ende eine Schneidkante ausgebildet ist sowie ein Greifinstrument mit einem Schaft, an dessen distalem Ende ein aus mindestens zwei Maulteilen bestehendes Werkzeug und an dessen proximalem Ende eine aus mindestens zwei Griffteilen bestehende Handhabe zur Betätigung des Werkzeugs angeordnet ist, wobei das Greifinstrument in Längsrichtung des Morcellatorschaftes verschiebbar in den hohlen Schaft des Morcellators einsetzbar ist.

Bei endoskopisch chirurgischen Eingriffen, wie beispielsweise bei der Laparoskopie, ist es üblich mehrere Zugänge zum Operationsgebiet zu schaffen, um dem Operationsgebiet über mindestens einen Zugang medizinische Instrumente, wie beispielsweise Schneid- und/oder Greifinstrumente, zur Durchführung der Operation und über mindestens einen weiteren Zugang eine Beobachtungseinheit, beispielsweise ein Endoskop, zur Beobachtung und Führung der Operation zuzuführen.

Wenn das Operationsgebiet aber räumlich sehr begrenzt und klein ist, wie dies beispielsweise bei Operationen in der Blase der Fall ist, ist es nicht möglich, die Beobachtungseinheit über einen separaten Zugang dem Operationsgebiet zuzuführen.

Für diese Zwecke ist es beispielsweise aus der DE 202 04 691 U1 bekannt, die optische Beobachtungseinheit zusammen mit einem Morcellator in den hohlen Schaft eines weiteren medizinischen Instruments, beispielsweise eines Resektoskops, einzusetzen. Bei dieser bekannten Instrumentenanordnung ist die aus einem Sichtkanal und Lichtleitern bestehende optische Beobachtungseinheit sichelförmig aufgebaut an der Innenwand des Resektoskopschaftes anliegend im Resektoskop angeordnet, um ein möglichst großen Arbeitskanal für den ebenfalls in den hohlen Resektoskopschaft eingesetzten Morcellator zu schaffen. Diese bekannte Instrumentenanordnung hat sich in der Praxis zwar bewährt, jedoch bedingen die Anordnung der optischen Beobachtungseinheit sowie des Morcellators im Resektoskopschaft einen kleinen Durchmesser der Morcellatorschneidkante. Nachteilig bei dieser bekannten Anordnung ist somit, dass die Morcellation mit einer kleinen Schneidkante bei großen zu morcellierenden Gewebebereichen sehr lange dauert.

Weiterhin ist aus der WO 99/07295 A1 ist ein System zur chirurgischen Entfernung von Gewebe bekannt, das aus einem Morcellator und einem in den hohlen Schaft des Morcellators einsetzbaren Greifinstrument besteht. zur Operation wird der Morcellator direkt in die künstliche Körperöffnung eingesetzt, so dass die äußere Mantelfläche des Morcellatorschaftes gegen den Patienten abdichtet.

Der Oberbegriff des Anspruchs 1 basiert auf diesem Dokument.

Davon ausgehend liegt der Erfindung die **Aufgabe** zugrunde, eine aus einem Morcellator und einem Greifinstrument bestehendes chirurgisches Instrumentensystem zu schaffen, das bei einfachem Aufbau vielseitig einsetzbar ist.

Die **Lösung** dieser Aufgabenstellung ist erfindungsgemäß dadurch gekennzeichnet, dass der Morcellator mit einsetzbarem Greifinstrument in einen hohlen Schaft eines weiteren medizinischen Instruments, insbesondere eines Resektoskops, einsetzbar ist, und dass ein zusätzlicher Spül- und/oder Saugkanal zwischen dem Schaft des Morcellators und der Innenseite des hohlen Schaftes des weiteren medizinischen Instruments ausgebildet ist.

Durch die erfindungsgemäße Verwendung eines weiteren Hohlschaftinstruments, in das der Morcellator samt Greifinstrument einsetzbar ist, lässt sich der Einsatzbereich des Instrumentensystems deutlich erweitern, da jetzt auch konstruktive Wechselwirkungen zwischen dem Morcellator und dem weiteren Hohlschaftinstrument zum Einsatz kommen können. Dieser zusätzliche Kanal dient im praktischen Betrieb vorzugsweise als Rückflusskanal.

Das solchermaßen ausgebildete chirurgische Instrumentensystem zeichnet sich somit dadurch aus, dass durch die konstruktive Ausgestaltung des Morcellatorschaftes und/oder der Innenseite des hohlen Schaftes des weiteren medizinischen Instruments ein zusätzlicher Spül- und/oder Saugkanal zwischen dem Schaft des Morcellators und der Innenseite des hohlen Schaftes des weiteren medizinischen Instruments ausbildbar ist, der einen verbesserten Einsatz des erfindungsgemäßen chirurgischen Instrumentensystems ermöglicht.

Zur Ausbildung des zusätzlichen Spül- und/oder Saugkanals ist gemäß einer ersten praktischen Ausführungsform das distale Ende des hohlen Schaftes des weiteren medizinischen Instruments nach innen gebogen ausgebildet. Das Umbiegen des distalen Endes des hohlen Schaftes ist zudem vorteilhaft, um ein atraumatisches Einführen des Schaftes in das Operationsgebiet zu gewährleisten.

Mit einer zweiten Ausführungsform der Erfindung wird vorgeschlagen, dass zur Ausbildung des zusätzlichen Spül- und/oder Saugkanals in der Mantelfläche des Schaftes des Morcellators mindestens eine Längsnut ausgebildet ist.

Der zusätzliche Spül- und/oder Saugkanal kann gemäß einer dritten praktischen Ausführungsform der Erfindung dadurch ausgebildet werden, dass der Schaft des Morcellators beabstandet von der distalseitigen Schneidkante einen den Durchmesser des Schaftes verringernden Kalibersprung aufweist.

Zur proximalseitigen Abdichtung des zusätzlichen Spül- und/oder Saugkanals wird vorgeschlagen, dass der zusätzliche Spül- und/oder Saugkanal über eine Dichtung, insbesondere eine Dichtung aus Teflon®, gegenüber der Innenseite des hohlen Schaftes des weiteren medizinischen Instruments abgedichtet ist.

Weiterhin wird mit der Erfindung vorgeschlagen, dass das Greifinstrument zusätzlich mit einer optischen Beobachtungseinheit sowie mindestens einem Spül- und/oder Saugkanal ausgestattet ist, wobei der mindestens eine Spül- und/oder Saugkanal innerhalb der optischen Beobachtungseinheit angeordnet ist. Durch das Verlagern der optischen Beobachtungseinheit und mindestens eines Spül- und/oder Saugkanals an das Greifinstrument und somit in das Innere des Morcellatorschaftes wird die Bewegungsfreiheit des Morcellators erhöht und die Möglichkeit gegeben, den Morcellatordurchmesser und somit die Schneidengröße zu vergrößern.

Da es beim Einführen des Morcellators in das Operationsgebiet passieren kann, dass nicht zu behandelndes Gewebe mit der Schneidkante des Morcellatorschaftes in Kontakt kommt und durch die scharfe Schneidkante verletzt wird, ist gemäß einer weiteren Ausgestaltungsform der Erfindung am Greifinstrument ein vorzugsweise als verdickter Bereich ausgebildeter Schneidkantenschutz vorgesehen, der zumindest an der Schneidkante des Morcellators formschlüssig, das heißt radial bündig an der Innenseite des Schaftes des Morcellators anliegt. Durch dieses formschlüssige Anliegen an der Innenseite der Schneidkante und das in axialer Richtung zumindest bündige Abschließen des Schneidkantenschutzes mit der Schneidkante werden Verletzungen beim Einführen des Morcellators verhindert.

Vorzugsweise ist dieser als Schneidkantenschutz dienende verdickte Bereich proximalseitig mit Abstand zum Werkzeug am Schaft des Greifinstruments angeordnet.

Gemäß einer bevorzugten Ausführungsform der Erfindung ist das in den Morcellatorschaft einsetzbare Greifwerkzeug eine mit zwei verschwenkbaren Maulteilen versehene Fasszange zum Ergreifen und Entfernen von morcelliertem Gewebe.

Um die Handhabung des Greifwerkzeugs für den Operateur zu erleichtern, wird mit der Erfindung weiterhin vorgeschlagen, dass die Maulteile in ihrer Stellung zueinander fixierbar sind, so dass der Operateur beispielsweise die Griffteile der Handhabe freigeben kann, ohne dabei das von den Maulteilen erfasste Gewebeteil zu verlieren.

Das Fixieren der Maulteile zueinander kann dabei entweder in vorgegebenen Winkelstellungen oder aber auch völlig frei wählbar erfolgen, wobei das mechanische Fixieren der Maulteile über die Handhabe, die Maulteile selbst oder über eine die Maulteile und die Handhabe miteinander verbindende Schub-/Zugstange erfolgen kann.

Um zu verhindern, dass die Maulteile des in der Greifstellung distalseitig außerhalb des Morcellatorschaftes arbeitenden Werkzeugs beim Hereinziehen des Greifwerkzeugs in den Morcellatorschaft gegen die Schneidkante des Morcellatorschaftes anlaufen und diese beschädigen, wird gemäß einer praktischen Ausführungsform der Erfindung vorgeschlagen, dass der Verschwenkwinkel der Maulteile zueinander beim Einziehen des Greifwerkzeugs in den Morcellatorschaft derart begrenzbar ist, dass die Maulteile des Werkzeugs berührungsfrei in den Schaft einziehbar sind.

Schließlich wird mit der Erfindung vorgeschlagen, dass der hohle Schaft des Morcellators über eine Doppeldichtungsanordnung gegenüber der Umgebung abdichtbar ist, wobei vorzugsweise eine Dichtung der Doppeldichtungsanordnung als Ringdichtscheibe zum Abdichten gegenüber dem in den Schaft einführbaren Greifinstrument ausgebildet ist und die andere Dichtung der Doppeldichtungsanordnung vorzugsweise als Kreuzschlitzdichtung ausgebildet ist, die das Innenlumen des Schaftes bei herausgezogenem Greifinstrument abdichtet.

Weitere Merkmale und Vorteile der Erfindung ergeben sich anhand der Beschreibung der zugehörigen Zeichnung, in der zwei Ausführungsbeispiele eines erfindungsgemäßen chirurgischen Instrumentensystems nur beispielhaft dargestellt sind. In der Zeichnung zeigt:
- Fig. 1: eine schematische Seitenansicht eines in ein Resektoskop eingesetzten erfindungsgemäßen chirurgischen Instrumentensystems;
- Fig. 2: einen vergrößerten schematischen Querschnitt entlang der Schnittlinie II-II gemäß Fig. 1 mit in den Morcellatorschaft eingesetztem Greifinstrument;
- Fig. 3: einen vergrößerten ausschnittweisen Längsschnitt entlang der Schnittlinie III-III gemäß Fig. 1 durch den Resektoskopschaft mit eingesetztem Morcellatorschaft, jedoch ohne Greifinstrument, eine erste erfindungsgemäße Ausführungsform darstellend;
- Fig. 4: einen Längsschnitt gemäß Fig. 3, jedoch zweite erfindungsgemäße Ausführungsform darstellend und
- Fig. 5: eine schematische Seitenansicht einer alternativen Ausführungsform eines in ein erfindungsgemäßes chirurgisches Instrumentensystem einsetzbaren Greifinstruments.

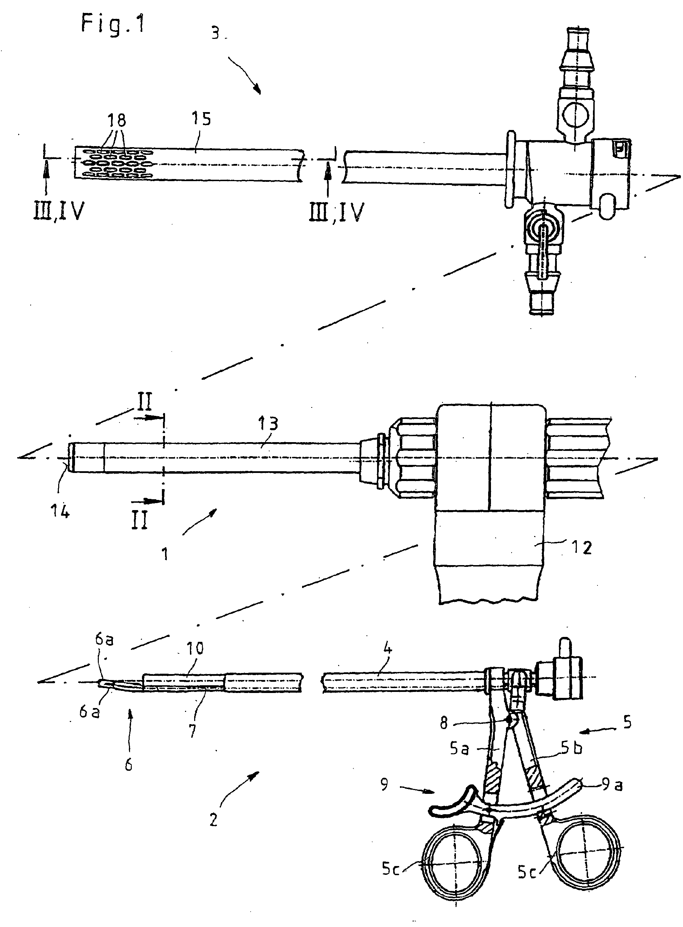

Das in der Abbildung Fig. 1 dargestellte chirurgische Instrumentensystem besteht aus einem Morcellator 1, einem in den Morcellator 1 einsetzbaren Greifinstrument 2 sowie einem Resektoskop 3 zur Aufnahme des mit dem Greifinstrument 2 bestückten Morcellators 1.

Das als Fasszange ausgebildete Greifinstrument 2 besteht im wesentlichen aus einem hohlen Schaft 4, an dessen proximalem Ende eine Handhabe 5 angeordnet ist, die aus einem starren Griffteil 5a und einem gegenüber dem starren Griffteil 5a verschwenkbaren Griffteil 5b besteht. Am distalen Ende des Schaftes 4 ist ein Werkzeug 6 angeordnet, welches beim dargestellten Ausführungsbeispiel zwei gegeneinander verschwenkbare Maulteile 6a aufweist. Zur Betätigung des Werkzeugs 6 sind die Handhabe 5 und das die Maulteile 6a des Werkzeugs 6 über eine im Inneren des Schaftes gelagerte Schub-/Zugstange 7 miteinander verbunden.

Zum sicheren Ergreifen der Griffteile 5a, 5b der Handhabe 5 weisen diese an ihren freien Enden Fingerösen 5c auf. Bei der dargestellten Ausführungsform lassen sich die um eine Schwenkachse 8 zueinander verschwenkbaren Griffteile 5a, 5b über einen Arretiermechanismus 9 in der verschwenkten Position fixieren. Über dieses Fixieren der Griffteile 5a, 5b zueinander werden unmittelbar auch die über die Schub-/Zugstange 7 mit den Griffteilen 5a, 5b verbundenen Maulteile 6a in ihrer entsprechenden Winkelstellung zueinander fixiert.

Der das Werkzeug 6 fixierende Arretiermechanismus 9 ermöglicht es dem Operateur ein von den Maulteilen 6a erfasstes Gewebeteil auch dann weiterhin sicher zu halten, wenn er die Handhabe 5 nicht mehr betätigt, wodurch seine Arbeit deutlich erleichtert wird.

Neben der dargestellten Ausführungsform, bei der der Arretiermechanismus 9 als am Griffteil 5a der Handhabe 5 angeordnete sichelförmige Raststange 9a ausgebildet ist, die mit nicht dargestellten Rastzähnen am Griffteil 5b der Handhabe 5 kämmt, ist es selbstverständlich auch möglich, einen Arretiermechanismus 9 im Bereich der Maulteile 6a oder der Schub-/Zugstange 7 auszubilden, um die Maulteile in ihrer Winkelstellung zueinander zu fixieren, wobei das Fixieren der Maulteile zueinander entweder in vorgegebenen Winkelstellungen oder aber auch völlig frei wählbar erfolgen kann.

Als Besonderheit weist das Greifinstrument 2 eine im Schaft 4 angeordnete optische Beobachtungseinheit 10 auf, über die das Arbeiten mit dem Werkzeug 6 direkt beobachtet und geführt werden kann. Die optische Beobachtungseinheit 10 selbst besteht aus mindestens einem Sichtkanal und mindestens einem Lichtleiter.

Zusätzlich zu der optischen Beobachtungseinheit 10 weist das Greifinstrument 2 mindestens einen im Schaft 4 angeordneten Spül- und/oder Saugkanal 11 auf, um im Operationsgebiet für gute Licht- und Sichtverhältnisse zu sorgen und ein übersichtliches Operieren zu ermöglichen. Der mindestens eine Spül- und/oder Saugkanal 11 ist an eine externe Spül-Saug-Einheit anschließbar.

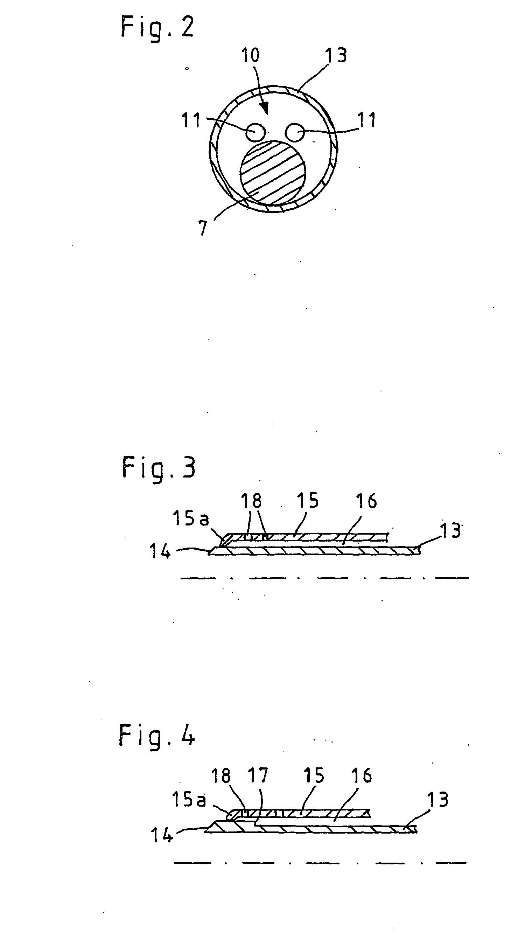

Wie aus der in Fig. 2 dargestellten Schnittdarstellung ersichtlich, sind bei dieser Ausführungsform zwei Spül- und/oder Saugkanäle 11 innerhalb der nicht näher dargestellten optischen Beobachtungseinheit 10 angeordnet.

Zur Aufnahme des mit dem Werkzeug 6, der optischen Beobachtungseinheit 10 sowie dem mindestens einen Spül- und/oder Saugkanal 11 versehenen Schaftes 4 des Greifinstruments 2 weist der über eine Handhabe 12 bedienbare Morcellator 1 einen hohlen zylindrischen Schaft 13 auf, an dessen distalem Ende eine Schneidkante 14 ausgebildet ist.

Das Greifinstrument 2 ist in Längsrichtung des Morcellatorschaftes 13 verschiebbar so in dem Morcellatorschaft 13 gelagert, dass die Maulteile 6a des Werkzeugs 6 in einer Greifstellung distalseitig aus dem Morcellatorschaft 13 herausragen und in einer Ruheposition im Inneren des Morcellatorschaftes 13 angeordnet sind. Die Kombination von Greifinstrument 2 und Morcellator 1 dient dazu zu morcellierendes Gewebe über das Greifinstrument 2 zu ergreifen und gegen die vorzugsweise um ihre Längsachse rotierende Schneidkante 14 des Morcellators 1 zu ziehen. Das solchermaßen morcellierte Gewebe kann nun mittels des Greifinstruments 2 in das Innere des Morcellatorschaftes 13 gezogen und durch Herausziehen des Greifinstruments 2 aus dem Morcellator 1 vollständig entfernt werden.

Dadurch, dass das Greifinstrument 2 zusätzlich mit einer optischen Beobachtungseinheit 10 versehen ist, kann diese optische Beobachtungseinheit 10 gleichzeitig dazu verwendet werden, den Morcelliervorgang zu beobachten und zu führen. Gerade bei räumlich begrenzten kleinen Operationsgebieten, wie beispielsweise in der Blase, ist eine solche Verlagerung der optischen Beobachtungseinheit 10 und zusätzlich mindestens eines Spül- und/oder Saugkanals 11 in den Morcellator 1 hinein vorteilhaft, da weitere Zugänge für separate Beobachtungs- und Spül-Saug-Einheiten meist nicht möglich sind.

Zum Einführen dieser aus dem Morcellator 1 und dem in den Morcellatorschaft 13 einsetzbaren Greifinstrument 2 bestehenden Anordnung medizinischer Instrumente in ein Operationsgebiet dient ein weiteres, mit einem hohlen Schaft 15 versehenes Instrument, wie beispielsweise das in der Darstellung wiedergegebene Resektoskop 3.

Wie aus den Schnittdarstellungen gemäß Fig. 3 und Fig. 4 ersichtlich, ist bei der Anordnung des Morcellators 1 innerhalb des hohlen Schaftes 15 des weiteren Instruments ein zusätzlicher, meist als Rückflusskanal dienender Spül- und/oder Saugkanal 16 zwischen dem Schaft 13 des Morcellators 1 und der Innenseite des hohlen Schaftes 15 des weiteren medizinischen Instruments ausbildbar.

Bei der in Fig. 3 dargestellten ersten Ausführungsform ist das distale Ende 15a des hohlen Schaftes 15 des weiteren medizinischen Instruments zur Ausbildung des zusätzlichen Spül- und/oder Saugkanals 16 nach innen gebogen ausgebildet.

Gemäß der in Fig. 4 dargestellten zweiten Ausführungsform weist der Schaft 13 des Morcellators 1 zur Ausbildung des zusätzlichen Spül- und/oder Saugkanals 16 beabstandet von der distalseitigen Schneidkante 14 einen den Durchmesser des Schaftes 13 verringernden Kalibersprung 17 auf.

Alternativ zu diesen beiden dargestellten Ausführungsformen ist es beispielsweise auch möglich, zur Ausbildung des zusätzlichen Spül- und/oder Saugkanals 16 in der Mantelfläche des Schaftes 13 des Morcellators 1 mindestens eine Längsnut auszubilden.

Damit Flüssigkeit über den zusätzlichen Spül- und/oder Saugkanal 16 vom Operationsgebiet fort abgeführt werden kann, sind im distalen Bereich des Schaftes 15 des weiteren medizinischen Instruments Ansaugöffnungen 18 ausgebildet, über die beispielsweise Blut und/oder Spülflüssigkeit in den zusätzlichen Spül- und/oder Saugkanal 16 gesaugt werden können.

Um einen Druckverlust über den zusätzlichen Spül- und/oder Saugkanal 16 zu vermeiden, ist der zusätzliche Spül- und/oder Saugkanal 16 proximalseitig über eine nicht dargestellte Dichtung, insbesondere eine Dichtung aus Teflon®, abgedichtet, die an der Innenseite des hohlen Schaftes 15 des weiteren medizinischen Instruments abdichtend anliegt.

Weitere, nicht dargestellte Dichtungen sind vorgesehen, um den hohlen Schaft 13 des Morcellators 1 gegenüber der Umgebung abzudichten. Diese Abdichtung erfolgt vorteilhafterweise über eine Doppeldichtungsanordnung, wobei vorzugsweise eine Dichtung der Doppeldichtungsanordnung als Ringdichtscheibe zum Abdichten gegenüber dem in den Schaft 13 einführbaren Greifinstrument 2 ausgebildet ist und die andere Dichtung der Doppeldichtungsanordnung vorzugsweise als Kreuzschlitzdichtung ausgebildet ist, die das Innenlumen des Schaftes 13 bei herausgezogenem Greifinstrument 2 abdichtet.

Damit beim Einführen des mit dem Greifinstrument 2 bestückten Morcellators 1 über das Resektoskop 3 in den Körper des Patienten nicht zu behandelndes Gewebe nicht durch die scharfe Schneidkante 14 des Morcellators 1 verletzt wird, sobald der Morcellator 1 distalseitig aus dem Schaft 15 des Resektoskops 3 heraustritt, weist der Schaft 4 des Greifinstruments 2 im wesentlichen auf der gesamten Länge des Schaftes 4 einen Außendurchmesser auf, der dem Innendurchmesser des Morcellatorschaftes 13 an der Schneidkante 14 entspricht. Durch dieses formschlüssige Anliegen des Schaftes 4 an der Innenseite der Schneidkante 14 und das in axialer Richtung zumindest bündige Abschließen des Schaftes 4 mit der Schneidkante 14 können Verletzungen beim Einführen des Morcellators 1 verhindert werden und kann die Schneidkante 14 vor eventuellen Beschädigungen geschützt werden.

Vor dem Einführen des Morcellators 1 in das Operationsgebiet muss das Greifinstrument 2 somit zunächst in Axialrichtung in den Morcellatorschaft 13 eingeschoben werden, bis es distalseitig zumindest bündig mit der Schneidkante 14 des Morcellators 1 abschließt, um die Schneidwirkung der Schneidkante 14 aufzuheben. Zumindest bündiges Abschließen mit der Schneidkante 14 bedeutet in diesem Zusammenhang, dass das distale Ende des Schaftes 4 vorteilhafterweise mit einem geringen Überstand die Schneidkante 14 distalseitig überragt, wodurch der Schneidkantenschutz verbessert wird. Um jederzeit die richtige Einschubtiefe des Schaftes 4 des Greifinstruments 2 in den Morcellatorschaft 13 zu gewährleisten, so dass der beschriebene Schneidkantenschutz wirksam werden kann, ist es weiterhin möglich, am Morcellator 1 und/oder am Greifinstrument 2 einen Anschlag auszubilden, der bei gegenseitigen Anlage der Instrumente 1 und 2 die richtige Einschubtiefe gewährleistet.

Um zu verhindern, dass die Maulteile 6a des in der Greifstellung distalseitig außerhalb des Morcellatorschaftes 13 arbeitenden Werkzeugs 6 beim Hereinziehen des Greifwerkzeugs 2 in den Morcellatorschaft 13 gegen die Schneidkante 14 des Morcellatorschaftes 13 anlaufen und diese beschädigen, ist der Verschwenkwinkel der Maulteile 6a zueinander beim Einziehen des Greifwerkzeugs 2 in den Morcellatorschaft 13 derart begrenzbar, dass die Maulteile 6a des Werkzeugs 6 berührungsfrei in den Schaft 13 einziehbar sind.

Ein solchermaßen ausgestaltetes chirurgisches Instrumentensystem zeichnet sich dadurch aus, dass sie bei einfachem Aufbau und vielseitiger Verwendbarkeit einen größtmöglichen Schneidkantendurchmesser des Morcellators 1 bei gleichzeitiger permanenter Sichtkontrolle über die optische Beobachtungseinheit 10 ermöglicht.

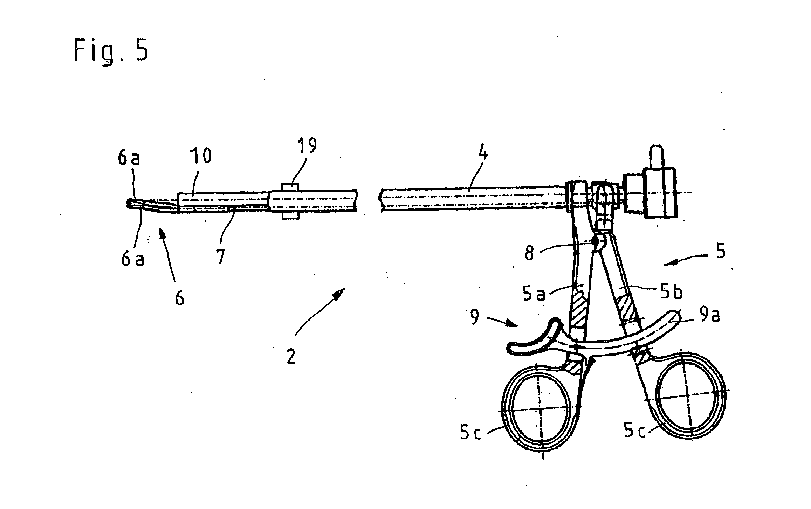

Fig. 5 zeigt eine alternative Ausführungsform des in den Morcellator 1 einschiebbaren Greifinstruments 2. Von dem in Fig. 1 dargestellten Greifinstrument unterscheidet sich dieses Greifinstrument durch die Verwirklichung des Schneidkantenschutzes. Damit beim Einführen des mit dem Greifinstrument 2 bestückten Morcellators 1 über das Resektoskop 3 in den Körper des Patienten nicht zu behandelndes Gewebe nicht durch die scharfe Schneidkante 14 des Morcellators 1 verletzt wird, sobald der Morcellator 1 distalseitig aus dem Schaft 15 des Resektoskops 3 heraustritt, ist bei dieser alternativen Ausführungsform am Schaft 4 des Greifinstruments 2 ein als verdickter Bereich 19 ausgebildeter Schneidkantenschutz vorgesehen, der zumindest an der Schneidkante 14 des Morcellators 1 formschlüssig an der Innenseite des Morcellatorschaftes 13 anliegt. Durch dieses formschlüssige Anliegen an der Innenseite der Schneidkante 14 und das in axialer Richtung zumindest bündige Abschließen des verdickten Bereichs 19 mit der Schneidkante 14 können Verletzungen beim Einführen des Morcellators 1 verhindert werden.

Bei der dargestellten Ausführungsform ist der verdickte Bereich 19 proximalseitig mit Abstand zum Werkzeug 6 am Schaft 4 des Greifinstruments 2 angeordnet. Vor dem Einführen des Morcellators 1 in das Operationsgebiet muss das Greifinstrument 2 somit zunächst in Axialrichtung in den Morcellatorschaft 13 eingeschoben werden, bis der verdickte Bereich 19 distalseitig zumindest bündig mit der Schneidkante 14 des Morcellators 1 abschließt, um die Schneidwirkung der Schneidkante 14 aufzuheben und vor eventuellen Beschädigungen zu schützen.

### Bezugszeichenliste

- 1: Morcellator
- 2: Greifinstrument
- 3: Resektoskop
- 4: Schaft
- 5: Handhabe
- 5a: starres Griffteil
- 5b: verschwenkbares Griffteil
- 5c: Fingeröse
- 6: Werkzeug
- 6a: Maulteil
- 7: Schub-/Zugstange
- 8: Schwenkachse
- 9: Arretiermechanismus
- 9a: Raststange
- 10: Beobachtungseinheit
- 11: Spül- und/oder Saugkanal
- 12: Handhabe
- 13: Schaft / Morcellatorschaft
- 14: Schneidkante
- 15: Schaft
- 15a: distales Ende
- 16: zusätzlicher Spül- und/oder Saugkanal
- 17: Kalibersprung
- 18: Ansaugöffnung
- 19: verdickter Bereich

## Patentansprüche

1. Chirurgisches Instrumentensystem, umfassend einen Morcellator (1) mit einem hohlen Schaft (13), an dessen distalem Ende eine Schneidkante (14) ausgebildet ist sowie ein Greifinstrument (2) mit einem Schaft (4), an dessen distalem Ende ein aus mindestens zwei Maulteilen (6a) bestehendes Werkzeug (6) und an dessen proximalem Ende eine aus mindestens zwei Griffteilen (5a, 5b) bestehende Handhabe (5) zur Betätigung des Werkzeugs (6) angeordnet ist, wobei das Greifinstrument (2) in Längsrichtung des Morcellatorschaftes (13) verschiebbar in den hohlen Schaft (13) des Morcellators (1) einsetzbar ist,
**dadurch gekennzeichnet,**
**dass** der Morcellator (1) mit einsetzbarem Greifinstrument (2) in einen hohlen Schaft (15) eines weiteren medizinischen Instruments, insbesondere eines Resektoskops (3), einsetzbar ist, und dass ein zusätzlicher Spül- und/oder Saugkanal (16) zwischen dem Schaft (13) des Morcellators (1) und der Innenseite des hohlen Schaftes (15) des weiteren medizinischen Instruments ausgebildet ist.

2. Chirurgisches Instrumentensystem nach Anspruch 1, **dadurch gekennzeichnet, dass** das distale Ende (15a) des hohlen Schaftes (15) des weiteren medizinischen Instruments zur Ausbildung des zusätzlichen Spül- und/oder Saugkanals (16) nach innen gebogen ausgebildet ist.

3. Chirurgisches Instrumentensystem nach Anspruch 1, **dadurch gekennzeichnet, dass** zur Ausbildung des zusätzlichen Spül- und/oder Saugkanals (16) in der Mantelfläche des Morcellatorschaftes (13) mindestens eine Längsnut ausgebildet ist.

4. Chirurgisches Instrumentensystem nach Anspruch 1, **dadurch gekennzeichnet, dass** der Schaft (13) des Morcellators (1) zur Ausbildung des zusätzlichen Spül- und/oder Saugkanals (16) beabstandet von der distalseitigen Schneidkante (14) einen den Durchmesser des Schaftes (13) verringernden Kalibersprung (17) aufweist.

5. Chirurgisches Instrumentensystem nach einem der Ansprüche 1 bis 4, **dadurch gekennzeichnet, dass** der zusätzliche Spül- und/oder Saugkanal (16) proximalseitig über eine Dichtung gegenüber der Innenseite des hohlen Schaftes (15) des weiteren medizinischen Instruments abgedichtet ist.

6. Chirurgisches Instrumentensystem nach einem der Ansprüche 1 bis 5, **dadurch gekennzeichnet, dass** das Greifinstrument (2) zusätzlich mit einer optischen Beobachtungseinheit (10) sowie mindestens einem Spül- und/oder Saugkanal (11) ausgestattet ist, wobei der mindestens eine Spül- und/oder Saugkanal (11) innerhalb der optischen Beobachtungseinheit (10) angeordnet ist.

7. Chirurgisches Instrumentensystem nach einem der Ansprüche 1 bis 6, **dadurch gekennzeichnet, dass** am Greifinstrument (2) ein Schneidkantenschutz ausgebildet ist, der beim Einführen des Morcellators (1) in ein Operationsgebiet des Morcellators (1) radial bündig mit der Schneidkante (14) des Morcellators (1) in Anlage bringbar ist und in axialer Richtung zumindest bündig mit der Schneidkante (14) abschließt.

8. Chirurgisches Instrumentensystem nach Anspruch 7, **dadurch gekennzeichnet, dass** der Schneidkantenschutz als proximalseitig mit Abstand zum Werkzeug (6) am Schaft (4) des Greifinstruments (2) angeordneter verdickter Bereich (19) ausgebildet ist.

9. Chirurgisches Instrumentensystem nach einem der Ansprüche 1 bis 8, **dadurch gekennzeichnet, dass** das Greifinstrument (2) eine mit zwei verschwenkbaren Maulteilen (6a) versehene Fasszange ist.

10. Chirurgisches Instrumentensystem nach Anspruch 9, **dadurch gekennzeichnet, dass** die Maulteile (6a) in ihrer Stellung zueinander fixierbar sind.

11. Chirurgisches Instrumentensystem nach Anspruch 10, **dadurch gekennzeichnet, dass** die Maulteile (6a) in vorgegebenen Winkelstellungen zueinander fixierbar sind.

12. Chirurgisches Instrumentensystem nach Anspruch 10 oder 11, **dadurch gekennzeichnet, dass** das Fixieren der Maulteile (6a) zueinander über die Handhabe (5) erfolgt.

13. Chirurgisches Instrumentensystem nach Anspruch 10 oder 11, **dadurch gekennzeichnet, dass** das Fixieren der Maulteile (6a) zueinander über die Maulteile (6a) erfolgt.

14. Chirurgisches Instrumentensystem nach Anspruch 10 oder 11, **dadurch gekennzeichnet, dass** das Fixieren der Maulteile (6a) zueinander über eine die Handhabe (5) mit den Maulteilen (6a) verbindende Schub-/Zugstange (7) erfolgt.

15. Chirurgisches Instrumentensystem nach einem der Ansprüche 1 bis 14, **dadurch gekennzeichnet, dass** der Verschwenkwinkel der Maulteile (6a) zueinander beim Einziehen des Greifwerkzeugs (2) in den Schaft (13) des Morcellators (1) derart begrenzbar ist, dass die Maulteile (6a) des Werkzeugs (6) berührungsfrei in den Schaft (13) einziehbar sind.

16. Chirurgisches Instrumentensystem nach einem der Ansprüche 1 bis 15, **dadurch gekennzeichnet, dass** der hohle Schaft (13) des Morcellators (1) über eine Doppeldichtungsanordnung gegenüber der Umgebung abdichtbar ist.

17. Chirurgisches Instrumentensystem nach Anspruch 16 **dadurch gekennzeichnet, dass** eine Dichtung der Doppeldichtungsanordnung als Ringdichtscheibe zum Abdichten gegenüber dem in den Morcellatorschaft (13) einführbaren Greifinstrument (2) ausgebildet ist und die andere Dichtung der Doppeldichtungsanordnung als Kreuzschlitzdichtung ausgebildet ist, die das Innenlumen des Morcellatorschaftes (13) bei herausgezogenem Greifinstrument (2) abdichtet.

## Claims

1. Surgical instrument system comprising a morcellator (1) with a hollow shaft (13), the distal end of which is formed as a cutting edge (14), and a gripping instrument (2) with a shaft (4), the distal end of which has a tool (6) consisting of at least two jaw parts (6a) and the proximal end of which has a handle (5) consisting of at least two gripping parts (5a, 5b) for operating the tool (6), the gripping instrument (2) being insertable into the hollow shaft (13) of the morcellator (1) by sliding it in along the longitudinal axis of the morcellator shaft (13), **characterized in that** the morcellator (1) with insertable gripping instrument (2) can be inserted into a hollow shaft (15) of a further medical instrument, in particular a resectoscope (3), and that an additional irrigation and/or suction channel (16) is formed between the shaft (13) of the morcellator (1) and the inner surface of the hollow shaft (15) of the further medical instrument.

2. Surgical instrument system according to Claim 1, **characterized in that** the distal end (15a) of the hollow shaft (15) of the further medical instrument curves inwards to form the additional irrigation and/or suction channel (16).

3. Surgical instrument system according to Claim 1, **characterized in that** the outer surface of the morcellator shaft (13) has at least one longitudinal groove to form the additional irrigation and/or suction channel (16).

4. Surgical instrument system according to Claim 1, **characterized in that** the shaft (13) of the morcellator (1) has an abrupt calibre change (17) - which reduces the diameter of the shaft (13) - at a distance from the distal cutting edge (14), to form the additional irrigation and/or suction channel (16).

5. Surgical instrument system according to one of Claims 1 to 4, **characterized in that** the additional irrigation and/or suction channel (16) is sealed off proximally via a seal against the inner surface of the hollow shaft (15) of the further medical instrument.

6. Surgical instrument system according to one of Claims 1 to 5, **characterized in that** the gripping instrument (2) is additionally equipped with an optical viewing unit (10) and at least one irrigation and/or suction channel (11), the at least one irrigation and/or suction channel (11) being arranged within the optical viewing unit (10).

7. Surgical instrument system according to one of Claims 1 to 6, **characterized in that** the gripping instrument (2) has a cutting-edge protector which, on introduction of the morcellator (1) into a surgical field of the morcellator (1), can be brought into contact radially flush with the cutting edge (14) of the morcellator (1) and in the axial direction closes at least flush with the cutting edge (14).

8. Surgical instrument system according to Claim 7, **characterized in that** the cutting-edge protector is formed as a thickened area (19) sited proximally, at a distance from the tool (6), on the shaft (4) of the gripping instrument (2).

9. Surgical instrument system according to one of Claims 1 to 8, **characterized in that** the gripping instrument (2) is a forceps provided with two pivotable jaw parts (6a).

10. Surgical instrument system according to Claim 9, **characterized in that** the jaw parts (6a) can be fixed in position with respect to each other.

11. Surgical instrument system according to Claim 10, **characterized in that** the jaw parts (6a) can be fixed in position with respect to each other at predetermined angle settings.

12. Surgical instrument system according to Claim 10 or 11, **characterized in that** the jaw parts (6a) are fixed in position with respect to each other via the handle (5).

13. Surgical instrument system according to Claim 10 or 11, **characterized in that** the jaw parts (6a) are fixed in position with respect to each other via the jaw parts (6a).

14. Surgical instrument system according to Claim 10 or 11, **characterized in that** the jaw parts (6a) are fixed in position with respect to each other via a push/pull rod (7) that connects the handle (5) to the jaw parts (6a).

15. Surgical instrument system according to one of Claims 1 to 14, **characterized in that** the angle of pivot between the jaw parts (6a) can be limited in such a way that, when the gripping tool (2) is introduced into the shaft (13) of the morcellator (1), the jaw parts (6a) of the tool (6) can be introduced into the shaft (13) without making contact.

16. Surgical instrument system according to one of Claims 1 to 15, **characterized in that** the hollow shaft (13) of the morcellator (1) can be sealed with respect to the environment via a double-seal arrangement.

17. Surgical instrument system according to Claim 16, **characterized in that** one seal of the double-seal arrangement is formed as an annular sealing disc to provide a seal with respect to the gripping instrument (2) which is insertable into the morcellator shaft (13), and the other seal of the double-seal arrangement is formed as a cross-slit seal which seals off the inner lumen of the morcellator shaft (13) when the gripping instrument (2) has been withdrawn.

## Revendications

1. Système d'instrument chirurgical, comportant un morcellateur (1) avec une tige creuse (13) sur l'extrémité distale de laquelle est réalisée une arête coupante (14) ainsi qu'un instrument de saisie (2) avec une tige (4) sur l'extrémité distale de laquelle est disposé un outil (6) composé d'au moins deux parties de mors (6a) et sur l'extrémité proximale de laquelle est disposée une poignée (5) composée d'au moins deux parties de poignée (5a, 5b) pour l'actionnement de l'outil (6), l'instrument de saisie (2) pouvant être inséré, de manière à pouvoir être déplacé dans le sens longitudinal de la tige du morcellateur (13), dans la tige creuse (13) du morcellateur (1),
**caractérisé en ce que** le morcellateur (1) peut être inséré avec un instrument de saisie (2) insérable dans une tige creuse (15) d'un autre instrument médical, en particulier d'un résectoscope (3) et **en ce qu'**un canal de rinçage et/ou d'aspiration (16) supplémentaire est réalisé entre la tige (13) du morcellateur (1) et la face interne de la tige creuse (15) de l'autre instrument médical.

2. Système d'instrument chirurgical selon la revendication 1, **caractérisé en ce que** l'extrémité distale (15a) de la tige creuse (15) de l'autre instrument médical est réalisée, pour la formation du canal supplémentaire de rinçage et/ou d'aspiration (16), recourbée vers l'intérieur.

3. Système d'instrument chirurgical selon la revendication 1, **caractérisé en ce que**, pour la formation du canal supplémentaire de rinçage et/ou d'aspiration (16), au moins une rainure longitudinale est réalisée dans la surface enveloppante de la tige du morcellateur (13).

4. Système d'instrument chirurgical selon la revendication 1, **caractérisé en ce que** la tige (13) du morcellateur (1) comporte, pour la formation du canal supplémentaire de rinçage et/ou d'aspiration (16), un changement de diamètre (17) rétrécissant le diamètre de la tige (13), à distance de l'arête coupante (14) située côté distal.

5. Système d'instrument chirurgical selon l'une quelconque des revendications 1 à 4, **caractérisé en ce que** le canal supplémentaire de rinçage et/ou d'aspiration (16) est étanchéifié, côté proximal, par le biais d'un joint par rapport à la face interne de la tige creuse (15) de l'autre instrument médical.

6. Système d'instrument chirurgical selon l'une quelconque des revendications 1 à 5, **caractérisé en ce que** l'instrument de saisie (2) est en outre équipé d'une unité d'observation optique (10) ainsi que d'au moins un canal de rinçage et/ou d'aspiration (11), le canal de rinçage et/ou d'aspiration (11) minimal étant disposé à l'intérieur de l'unité d'observation optique (10).

7. Système d'instrument chirurgical selon l'une quelconque des revendications 1 à 6, **caractérisé en ce que**, sur l'instrument de saisie (2), est réalisée une protection d'arête coupante qui peut être amenée, lors de l'introduction du morcellateur (1) dans une zone d'opération du morcellateur (1), à ras de l'arête coupante (14) du morcellateur (1) dans le sens radial et se termine au moins à ras de l'arête coupante (14) dans le sens axial.

8. Système d'instrument chirurgical selon la revendication 7, **caractérisé en ce que** la protection d'arête coupante est réalisée sous la forme d'une zone épaissie (19) disposée côté proximal à distance de l'outil (6) sur la tige (4) de l'instrument de saisie (2).

9. Système d'instrument chirurgical selon l'une quelconque des revendications 1 à 8, **caractérisé en ce que** l'instrument de saisie (2) est une pince à séquestre munie de deux parties de mors (6a) pouvant être pivotées.

10. Système d'instrument chirurgical selon la revendication 9, **caractérisé en ce que** les parties de mors (6a) peuvent être fixées l'une par rapport à l'autre dans leur position.

11. Système d'instrument chirurgical selon la revendication 10, **caractérisé en ce que** les parties de mors (6a) peuvent être fixées l'une par rapport à l'autre dans des positions angulaires prédéfinies.

12. Système d'instrument chirurgical selon la revendication 10 ou 11, **caractérisé en ce que** la fixation des parties de mors (6a) l'une par rapport à l'autre se fait par le biais de la poignée (5).

13. Système d'instrument chirurgical selon la revendication 10 ou 11, **caractérisé en ce que** la fixation des parties de mors (6a) l'une par rapport à l'autre se fait par le biais des parties de mors (6a).

14. Système d'instrument chirurgical selon la revendication 10 ou 11, **caractérisé en ce que** la fixation des parties de mors (6a) l'une par rapport à l'autre se fait par le biais d'une barre de poussée/traction (7) raccordant la poignée (5) aux parties de mors (6a).

15. Système d'instrument chirurgical selon l'une quelconque des revendications 1 à 14, **caractérisé en ce que** l'angle de pivotement des parties de mors (6a) l'une par rapport à l'autre peut être délimité, lors de l'insertion de l'outil de saisie (2) dans la tige (13) du morcellateur (1), de telle sorte que les parties de mors (6a) de l'outil (6) puissent être insérées sans contact dans la tige (13).

16. Système d'instrument chirurgical selon l'une quelconque des revendications 1 à 15, **caractérisé en ce que** la tige creuse (13) du morcellateur (1) peut être étanchéifiée, par rapport à l'environnement, par le biais d'un dispositif à double joint.

17. Système d'instrument chirurgical selon la revendication 16, **caractérisé en ce qu'**un joint du dispositif à double joint est réalisé sous la forme de disque d'étanchéité annulaire pour l'étanchéification par rapport à l'instrument de saisie (2) insérable dans la tige du morcellateur (13) et l'autre joint du dispositif à double joint est réalisé sous forme de joint à fente en croix qui étanchéifie la lumière interne de la tige du morcellateur (13) lorsque l'instrument de saisie (2) est retiré.
